Europäisches Patentamt

European Patent Office

Office européen des brevets

(19)

(11) Publication number: **0 308 099**

**A1**

## EUROPEAN PATENT APPLICATION

(12)

(21) Application number: 88307997.2

(22) Date of filing: 30.08.88

(51) Int. Cl.⁴: **C07C 2/66 , B01J 29/28**

(30) Priority: 02.09.87 US 92504

(43) Date of publication of application:
**22.03.89 Bulletin 89/12**

(84) Designated Contracting States:
**BE DE FR GB IT NL**

(71) Applicant: **MOBIL OIL CORPORATION**
**150 East 42nd Street**
**New York New York 10017(US)**

(72) Inventor: **Chu, Yung-Feng**
**4 Parker Road**
**Plainsboro New Jersey 08536(US)**
Inventor: **Marler, David Owen**
**801 Cooper Street, No. 272B**
**Deptford New Jersey 08096(US)**
Inventor: **McWilliams, John Paul**
**117 South American Street**
**Woodbury New Jersey 08096(US)**

(74) Representative: **Colmer, Stephen Gary**
**Patent Department c/o Mobil Services**
**Company Limited Mobil Court 3 Clements**
**Inn**
**London WC2A 2EB(GB)**

(54) **Alkylation of aromatics.**

(57) Alkylation of aromatics, e.g. ethylbenzene preparation, with minimized o-xylene make by contacting with olefin alkylating agent in the presence of ZSM-5 crystals having a diffusion rate constant of at least about 100 sec⁻¹ and an alpha value of less than 100.

EP 0 308 099 A1

## ALKYLATION OF AROMATICS

This invention relates to a process for the alkylation of an aromatic hydrocarbon by reaction with an olefin in the presence of a porous crystalline silicate ZSM-5 catalyst.

Alkylation of aromatic hydrocarbons utilizing porous crystalline silicate catalysts has heretofore been described. U.S. Pat. No. 2,904,607 to Mattox refers to alkylation of aromatic hydrocarbons with an olefin in the presence of a crystalline metallic aluminosilicate having uniform pore openings of about 6 to 15 Angstrom units. U.S. Pat. No. 3,251,897 to Wise describes liquid phase alkylation in the presence of X- or Y-type crystalline aluminosilicate zeolite, specifically such type zeolites wherein the cation is rare earth and/or hydrogen. U.S. Pat. No. 3,751,504 to Keown et al., and U.S. Pat. No. 3,751,506 to Burress describe vapor phase alkylation of aromatic hydrocarbons with olefins, e.g., benzene with ethylene in the presence of ZSM-5 zeolite catalyst. U.S. Pat. No. 4,016,218 to Wise describes vapor phase alkylation with a shape-selective zeolite such as ZSM-5 which has been modified by steaming to an alpha value less than 250.

While the latter type catalysts represent a distinct improvement over previously suggested crystalline aluminosilicate catalysts particularly with respect to improved aging properties, they have the disadvantage of producing unwanted quantities of impurities along with the desired alkyl aromatic product, thereby decreasing the overall yield and selectivity for such product.

Thus, in the alkylation of benzene with ethylene, while desired ethylbenzene is the major product, small amounts of di- and possibly triethylbenzenes are always produced simultaneously with ethylbenzene, such amounts depending on the conversion of benzene to ethylbenzene. The polyethylbenzenes formed can be recycled to the alkylation zone, where they undergo transalkylation with benzene to produce more ethylbenzene. Alternatively, the polyethylbenzenes can be transalkylated with benzene in a separate reactor. The formation of polyethylbenzenes hence does not constitute an ultimate loss of the alkylating agent, ethylene. On the other hand, aromatic compounds other than ethylbenzene and polyethylbenzenes, that are formed during the alkylation reaction, generally referred to as by-products, result in an irreversible loss of ethylene and cause difficulties in the product purification. By-products produced during ethylation of benzene include, for example, toluene, xylenes, cumene, n-propylbenzene, ethyltoluene, butylbenzene and other $C_{10}$ + aromatics, the majority being $C_7$-$C_9$ aromatics. Production of o-xylene is especially undesirable in view of its relatively low commercial value. The formation of these by-products is increased when the benzene conversion to ethylbenzene is high. Due to the high exothermicity of the alkylation reaction, ethylbenzene synthesis is generally carried out in a multiplicity of reactors with interstage cooling and addition of ethylene to the various stages, the ethylbenzene concentration increasing in subsequent stages. Undesired by-products are accordingly formed in increasing amounts in the latter stages of the process.

In accordance with the present invention, there has been discovered a process for decreasing the selectivity for o-xylene while using a catalyst which affords a high yield of the alkylate of interest over a long and commercially attractive period of time.

The process comprises effecting alkylation of aromatic hydrocarbons by contacting the same with an olefin under conditions effective for accomplishing said alkylation, e.g., a reactor inlet temperature between about 302° C and 482° C (575° F and 900° F), preferably with a reactor bed temperature as much as 139° C (250° F) above the reactor inlet temperature, a pressure between atmospheric and 20,790 kPa (3000 psig), employing a mole ratio of hydrocarbon charge aromatic to olefin alkylating agent in the approximate range of 1:1 to 30:1 and a total feed weight hourly space velocity between 2 and 2000, in the presence of a catalyst having an alpha value below 100 preferably below 75, say from 30 to 65, comprising a porous crystalline silicate having the structure of ZSM-5 and having a diffusion rate constant ($D/r^2 \times 10^6$) of at least 100 $sec^{-1}$, preferably at least 150, say greater than from 200 $sec^{-1}$ where D = the diffusion coefficient ($cm^2$/sec) and r = the crystal radius (cm).

Hydrocarbon sorption and diffusion rate measurements have been found to be effective in characterizing and distinguishing between various ZSM-5 preparations. Diffusion parameters are calculated from sorption rates by assuming that the plane sheet model describes the diffusion process in ZSM-5. For every sorbate loading $Q/Q\infty$, where $Q\infty$ is the equilibrium sorbate loading, there is a $(Dt/r^2)^{1/2}$ value where D, t, and r are the diffusion coefficient ($cm^2$/sec), time (sec), and crystal radius (cm), respectively. Hence from the sorption time required to reach a given sorbate loading, $Q/Q\infty$, $D/r^2$ can be calculated directly. Graphical solutions for the plane sheet model given by J. Crank in "The mathematics of Diffusion", Oxford University Press, Ely House, London, 1967, are tabulated below:

| Q/Q∞ | $(Dt/r^2)^{1/2}$ |
|------|------------------|
| 0.05 | 0.044 |
| 0.10 | 0.088 |
| 0.20 | 0.173 |
| 0.30 | 0.267 |
| 0.40 | 0.353 |

Sorption measurements are carried out with a Dupont Instruments Thermogravimetric Analyzer Model 951 and Sage Instruments Syring Pump Model 355. Xylene sorption and diffusion measurements are made by heating a fresh 50 mg sample of hydrogen form zeolite in flowing nitrogen at 500°C to a constant weight. The temperature is reduced to 120°C, and either ortho- or para-xylene is delivered to the TGA module by means of the syring pump. The hydrocarbon is instantaneously vaporized and swept over the zeolite by a 60 cc/min nitrogen purge. Para-xylene is used to obtain equilibrium xylene capacity due to the slow equilibrium approach of ortho-xylene.

For present purposes, $D/r^2$ values are calculated at a sorbate loading of Q/Q∞ = 0.3. A sample calculation for determining $D/r^2$ is presented below for a zeolite that required Y minutes to reach a Q/Q∞ value of 0.3.

$$D/r^2 = [(0.267)^2/(Y \text{ min} \times 60 \text{ sec/min})]$$

The porous crystalline silicates used in the present process can be prepared from a non-gelling, non-organic forming mixture having a solids content of at least about 30, preferably at least about 35 weight percent comprising a silica source of precipitated silica having a particle size of 1 to 500 microns, a source of alkali metal and a source of water.

Preferred methods for preparing such materials allow for flexibility in the exact composition of the zeolite product. That is, by controlling the exact components which constitute the crystallization reaction mixture from which the zeolite is crystallized, control of the product composition is possible. In preparing zeolites by this process, an essential component of the zeolite crystallization reaction mixture is a silica precipitate, the particle size of which is in the range of 1 to 500 microns. Moreover, the silica precursor precipitation can be undertaken in a liquid reaction mixture to which no aluminum or alumina source has been added; this embodiment provides great flexibility in varying the Si:Al atomic ratio (or $SiO_2/Al_2O_3$ molar ratio) in the zeolite composition. It is noted that the zeolites of the present invention have the structure of ZSM-5 and should preferably have a $SiO_2/Al_2O_3$ molar ratio of less than about 40.

ZSM-5 is described in U.S. Pat. No. 3,702,886. The crystal structure of ZSM-5 as defined by the X-ray diffraction "fingerprint", establishes the identity of the specific crystalline zeolite material. The crystal structure of known zeolites may include gallium, boron, iron and chromium as framework elements, without changing its identification by the X-ray diffraction "fingerprint"; and these gallium-, boron-, iron- and chromium-containing silicates and aluminosilicates may be useful in some applications described herein.

The crystals of zeolite in a form substantially free of alkali metal, i.e., containing less than about 0.5 weight percent alkali metal, and characterized by an alpha value hereinafter described may then be subjected to thermal treatment, preferably in the presence of steam, to reduce the activity thereof, as expressed in terms of alpha value, as described herein, to less than about 100 and preferably less than about 75, say, between about 30 and about 65. Alternately, such treatment may be accomplished after the zeolite has been composited with inorganic oxide.

The alpha value reflects the relative activity of the catalyst with respect to a high activity silica-alumina cracking catalyst. To determine the alpha value as such term is used herein, n-hexane conversion is determined at about 427°C (800°F). Conversion is varied by variation in space velocity such that a conversion level of 10 to 60 percent of n-hexane is obtained and converted to a rate constant per unit volume of zeolite and compared with that of silica-alumina catalyst which is normalized to a reference activity of 538°C (1000°F). Catalytic activity of the catalysts are expressed as a multiple of this standard, i.e., the silica-alumina standard. The silica-alumina reference catalyst contains 10 weight percent $Al_2O_3$ and the remainder $SiO_2$. This method of determining alpha, modified as described above, is more fully described in the Journal of Catalysis, Vol. VI, pages 278-287, 1966.

Thermal treatment will depend on the nature of the atmosphere to which the zeolite is exposed. When such atmosphere is an inert gas, the minimum effective temperature will be 649°C (1200°F) and may extend up to 982°C (1800°F). When the treating atmosphere is steam, lower temperatures may be used extending from 260°C to 982°C (500°F to 1800°F) depending on the stream pressure, with the use of higher pressure requiring a lower temperature. This treatment is carried on for a period of time suficient to effect the desired reduction in alpha. Generally, such period will be between about 1/2 hour and 100 hours. Such thermal treatment can be carried out in any inert atmosphere such as air, nitrogen, carbon dioxide, carbon monoxide, hydrogen, flue gas, argon, methane, helium, oxygen and suitable mixtures thereof, but is preferably effected in an atmosphere containing steam. A steam treating atmosphere may be employed which is 100 percent steam or steam admixed with a gas which is substantially inert with respect to the zeolite. It is contemplated that the thermal treatment will generally be effected at atmospheric pressure but pressures ranging from sub-atmospheric to several hundred atmospheres may be employed. With the use of elevated pressure, temperatures in the lower region of the above-described range will usually be applicable in achieving the desired reduction in alpha value of the zeolite under treatment. Thus, it has been found, that at elevated steam pressure, the temperature of treatment can be reduced substantially to achieve the same degree of modification.

In the case of many catalysts, it is desired to incorporate the zeolite ZSM-5 hereby prepared with another material resistant to the temperatures and other conditions employed in certain organic conversion processes. Such matrix materials include active and inactive materials and synthetic or naturally occurring zeolites as well as inorganic materials such as clays, silica and/or metal oxides, e.g. alumina. The latter may be either naturally occurring or in the form of gelatinous precipitates, sols or gels including mixtures of silica and metal oxides. Use of a material in conjunction with the zeolite ZSM-5, i.e. combined therewith, which is active, may enhance the conversion and/or selectivity of the catalyst in certain organic conversion processes. Inactive materials suitably serve as diluents to control the amount of conversion in a given process so that products can be obtained economically without employing other means for controlling the rate or reaction. Frequently, crystalline silicate materials have been incorporated into naturally occurring clays, e.g. bentonite and kaolin. These materials, i.e. clays, oxides, etc., function, in part, as binders for the catalyst. It is desirable to provide a catalyst having good physical strength, because in petroleum refinery processing, the catalyst is often subjected to conditions, which tend to break the catalyst down into powder-like materials which cause problems in processing. Preferably, the porous crystalline silicate is combined in an amount between about 1 and about 99 weight percent in an inorganic oxide binder, e.g., alumina, preferably between about 50 and about 75 weight percent.

In addition to the foregoing materials, the zeolite ZSM-5 catalyst used herein can be composited with a porous matrix material such as silica-alumina, silica-magnesia, silica-zirconia, silica-thoria, silica-beryllia, silica-titania, as well as ternary compositions such as silica-alumina-thoria, silica-alumina-zirconia, silica-alumina-magnesia and silica-magnesia-zirconia. The matrix can be in the form of a cogel. A mixture of these components could also be used.

Exemplary of the hydrocarbons which may be alkylated by the process of the present invention are aromatic compounds such as benzenes, naphthalenes, anthracenes, and the like and substituted derivatives thereof; and alkyl substituted aromatics, e.g. toluene, xylene and homologs thereof.

In accordance with this invention the alkylating agents employed are olefinic hydrocarbons having from 2 to 20 carbon atoms such as ethylene, propylene, and dodecylene, preferably having from 2 to 10 carbon atoms.

Operating conditions employed in the process of the present invention will be dependent, at least in part, on the specific alkylation reaction being effected. Such conditions as temperature, pressure, space velocity and molar ratio of the reactants and the presence of inert diluents will have important effects on the process.

The process of this invention can be conducted such that alkylation of an aromatic hydrocarbon compound, exemplified by benzene, is carried out by contact in a reaction zone, such as, for example, a fixed bed of catalyst, under alkylation effective conditions, said catalyst being characterized as above-described and preferably hydrogen exchanged such that a predominate portion of its exchangeable cations are hydrogen ions. In general, it is contemplated that more than 50 percent and preferably more than 75 percent of the cationic sites of the crystalline zeolite, above-described, will be occupied by hydrogen ions. The alkylatable aromatic compound and olefinic hydrocarbon, e.g., ethylene are desirably fed to a first stage at an appropriate mole ratio of one to the other. The feed to such first stage is heated. After some reaction takes place, such as, for example, when about 80% of the olefinic hydrocarbon is consumed, the effluent of the first stage is cooled to remove heat of reaction and more olefinic hydrocarbon is added (second stage) to maintain the mole ratio of aromatic compound to olefinic hydrocarbon within the range established for the first stage. A plurality of reaction stages are possible for the process of this invention. It is generally desirable to provide cooling between reactor stages.

Considering vapor-phase alkylation of benzene with ethylene, the first stage mole ratio of benzene to ethylene may be in the range of about 1:1 to about 30:1. The first stage feed is heated to a reactor inlet temperature within the range of 302° to 482° (575°F to 900°F) at a pressure within the range of atmospheric to 20,790 kPa (3000 psig). Preferred inlet temperatures fall within the range of 316°C to 454°C (600°F to 850°F) and preferred pressures fall within the range of 276 kPa to 3210 kPa (25 psig to 450 psig). The repeating of reaction staging is carried out under the same conditions while maintaining an overall aromatic hydrocarbon, e.g., benzene, to olefinic alkylating agent, e.g. ethylene, mole ratio of about 1:1 to about 30:1, with a preferred range of about 2.5:1 to about 25:1.

It is noted that extremely high total feed space velocities are possible in the process of this invention, i.e. up to 2000 hr$^{-1}$. An important factor in the present process is, however, the weight hourly space velocity (WHSV) of the alkylating agent, e.g. ethylene. The alkylating agent WHSV to each of any alkylation reactor stages is maintained between about 1 and about 10 hr$^{-1}$. For the most desirable alkylating agent, i.e., ethylene, WHSV is within the range of about 2 to about 8 hr$^{-1}$. When the ethylene WHSV is maintained within the above limits, an economical cycle between regeneration of catalyst exists.

The process of this invention may be carried out as a batch-type, semi-continuous or continuous operation utilizing a fixed or moving bed catalyst system. A preferred embodiment entails use of fluidized catalyst zone wherein the reactants, e.g. benzene and ethylene, are passed concurrently or countercurrently through a moving fluidized bed of the catalyst. The fluidized catalyst after use is conducted to a regeneration zone wherein coke is burned from the catalyst is an oxygen-containing atmosphere, e.g. air, at an elevated temperature, after which the regenerated catalyst is recycled to the conversion zone for further contact with the benzene and ethylene reactants.

In another aspect, the present invention relates to a method for effecting alkylation of an aromatic hydrocarbon charge whereby o-xylene production is minimized which comprises contacting said aromatic hydrocarbon charge with an olefinic hydrocarbon alkylating agent under conditions effective for accomplishing said alkylation in the presence of a catalyst composition having an alpha value below 100. The catalyst composition comprises a porous crystalline silicate whose crystals have a diffusion rate constant (D/r$^2$ X 10$^6$) of at least 100 sec$^{-1}$.

The porous crystalline silicate employed can be aluminosilicate having a silica to alumina molar ratio of less than 40, preferably between 20 and 30. The alpha value of the catalyst composition can be adjusted to below 100, preferably below 75, say from 30 to 65, by steaming at a temperature of 260°C to 982°C (500°F to 1800°F), preferably between 399 and 649°C (750 and 1200°F). Preferably, the alkylation process of the present invention is effected in the vapor phase, the aromatic hydrocarbon is benzene and the olefinic hydrocarbon alkylating agent is ethylen.

The following examples will serve to illustrate the process of the invention without limiting the same.

Example 1 - Preparation of Small Crystal ZSM-5 From Forming Mixture Containing Organics

A 3.1 parts quantity, by weight, of n-propylamine was added to a mixture containing 1.1 parts sodium chloride, 0.2 parts ZSM-5 seeds, 0.2 parts dispersant (mixture of polymerized aryl and substituted benzoid alkyl sulfonic acids), 2.6 parts Al$_2$(SO$_4$)$_3$•14 H$_2$O, 7.0 parts 50% NaOH, 25.8 parts amorphous precipitated silica (HiSil 233), available from PPG Industries, Chemical Division, and 59.9 parts water. The reaction mixture had a composition, in mole ratios, of:

$$SiO_2/Al_2O_3 = 65$$
$$H_2O/SiO_2 = 9.92$$
$$CH^-/SiO_2 = 0.163$$
$$N/Al_2O_3 = 9.2$$
$$OH^-/H_2O = 0.0165$$

wherein N is the n-propylamine. The hydroxide concentration is based on only inorganic sources.

The reaction mixture was then heated directly to 104°C (220°F) and stirred in an autoclave at that temperature for crystallization. After full crystallinity was achieved, the resulting crystals were separated from the remaining liquid by filtration, washed with water, exchanged with NH$_4$NO$_3$ and dried.

The resulting product had a SiO$_2$/Al$_2$O$_3$ molar ratio of 50, a diffusion rate constant (D/r$^2$ X 10$^6$) of greater than 150 sec$^{-1}$ and was composited with alumina binder to form a material having a ZSM-5 content of about 65 weight percent and an alpha of about 200. After steaming in 100% steam for 3 hour the alpha value was measured at 40.

Example 2 - Preparation of Small Crystal ZSM-5 from Non-Organic, Seeded Forming Mixture

A 7.3 parts quantity, by weight, of water was mixed with 12.8 parts 50% NaCH, 10.1 parts $Al_2(SO_4)_3 \bullet$ 14 $H_2O$, 1.6 parts ZSM-5 seeds and 68.2 parts amorphous silica (47.6% solids) prepared by the neutralization of sodium silicate with sulfuric acid. The reaction mixture had a composition, in mole ratios, of:

$SiO_2/Al_2O_3$ = 32
$H_2O/SiO_2$ = 5.45
$OH^-/SiO_2$ = 0.105
$OH^-/H_2O$ = 0.0192

The reaction mixture was then heated directly to 104°C (220°F) and stirred in an autoclave at that temperature for crystallization. After full crystallinity was achieved, the resulting crystals were separated from the remaining liquid by filtration, washed with water and dried.

The resulting product had a $SiO_2/Al_2O_3$ molar ratio of about 25, a diffusion rate constant $(D/r^2)$ greater than about 200 $sec^{-1}$ and was composited with alumina binder so that the resulting product had a ZSM-5 content of about 65 weight percent and an alpha value of about 500.

After 8 hours of steaming in 100% steam at 538°C (1000°F), the alpha value was reduced to 40.

Example 3 - Ethylation of Benzene Using ZSM-5

The catalysts of Examples 1 and 2 were evaluated in a fixed bed reactor for the ethylbenzene reaction. The result is shown in Table 2. It is clear that under similar conditions and ethylene conversion, the by-product formation was similar for the two catalysts except the o-xylene selectivity. The yield of o-xylene using the catalysts of Example 2 was reduced by half.

Table 2

| Feed: Benzene, 380 cc/hr; Ethylene, 160 cc/min Temperature: 399°C (750°F) WHSV: 4 (based on ethylene) Pressure: 2170 kPa (300 psig) | | |
|---|---|---|
| Catalyst | Example 1 | Example 2 |
| Ethylene Conversion | 98% | 98% |
| DIEB/EB | 0.082 | 0.086 |
| O-Xyl/EB X $10^{-4}$ | 1.8 | 0.9 |
| Xyl/EB X $10^{-4}$ | 11.6 | 12.9 |
| $C_9$ +/EB | 0.086 | 0.098 |
| where DIEB is diethylbenzene, EB is ethylbenzene, o-xyl is ortho-xylene and xyl is xylene. | | |

## Claims

1. A method for effecting alkylation of an aromatic hydrocarbon charge whereby o-xylene production is minimized which comprises contacting the aromatic hydrocarbon charge with an olefinic hydrocarbon alkylating agent under conditions effective for accomplishing alkylation in the presence of a catalyst composition having an alpha value below 100 which comprises a porous crystalline silicate ZSM-5 and whose crystals have a diffusion rate constant ($D/r^2 \times 10^6$) of at least about 100 sec$^{-1}$ where D = diffusion coefficient (cm$^2$/sec) and r = crystal radius (cm) of the crystalline silicate.

2. The method of claim 1 wherein the conditions include a reactor inlet temperature between 302°C and 482°C (575°F and 900°F), a reactor pressure between atmospheric and 20,790 kPa (3000 psig), employing a mole ratio of hydrocarbon aromatic charge to olefinic hydrocarbon alkylating agent in the approximate range of 1:1 to 30:1 and a total weight hourly space velocity between 2 and 2000 and the diffusion rate constant is at least 175 sec$^{-1}$.

3. The method of claim 2 wherein the alkylating agent is an olefinic hydrocarbon containing from 2 to 20 carbon atoms.

4. The method of claim 3 wherein the porous crystalline silicate is an aluminosilicate having a silica to alumina molar ratio of less than 40 and the diffusion rate constant is at least 200 sec$^{-1}$.

5. The method of claim 4 wherein the alpha value is adjusted to within the range of 30 to 65 by steaming at a temperature of 260°C to 982°C (500°F to 1800°F).

6. The method of claim 5 wherein the composition comprises porous crystalline silicate in an amount between 50 and 75 weight percent and an inorganic oxide binder.

7. The method of claim 6 wherein the binder is alumina.

8. The method of claim 7 wherein the alkylation is effected in the vapor phase, the aromatic hydrocarbon charge comprises benzene and the olefinic hydrocarbon alkylating agent comprises ethylene.

9. The method of claim 8 wherein the reactor inlet temperature is between 316°C and 454°C (600°F and 850°F) and the reactor pressure is between 276 kPa and 3210 kPa (25 and 450 psig).

10. The method of claim 3 wherein the silica to alumina molar ratio ranges between 20 and 40.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-4 016 218 (HAAG et al.)<br>----- | | C 07 C 2/66<br>B 01 J 29/28 |

TECHNICAL FIELDS
SEARCHED (Int. Cl.4)

C 07 C 2/66

The present search report has been drawn up for all claims

| Place of search | Date of completion of the search | Examiner |
|---|---|---|
| THE HAGUE | 11-01-1989 | VAN GEYT J.J.A. |

EPO FORM 1503 03.82 (P0401)